# EUROPÄISCHE PATENTSCHRIFT

(11) **EP 0 563 069 B1**
(45) Veröffentlichungstag und Bekanntmachung des Hinweises auf die Patenterteilung: **17.04.1996**
(21) Anmeldenummer: 91920713.4
(22) Anmeldetag: 03.12.1991
(51) Int. Cl.: C07C 68/06, C07C 69/96, C10M 107/02

(54) **GUERBETCARBONATE**
GUERBET CARBONATES
CARBONATES DE GUERBET

(30) Priorität: 15.12.1990 DE 4040154
(43) Veröffentlichungstag der Anmeldung: 06.10.1993
(73) Patentinhaber: Henkel Kommanditgesellschaft auf Aktien, D-40191 Düsseldorf (DE)
(72) Erfinder: WESTFECHTEL, Alfred, D-4010 Hilden (DE); BONGARDT, Frank, D-4000 Düsseldorf 13 (DE); ANSMANN, Achim, D-4006 Erkrath (DE)
(86) Internationale Anmeldenummer: EP9102292
(87) Internationale Veröffentlichungsnummer: WO9210462

(56) Entgegenhaltungen:
- EP-A- 0 437 764
- DE-A- 2 335 728
- GB-A- 1 129 229
- US-A- 4 395 370
- US-A- 4 447 365
- US-A- 4 522 765
- US-A- 4 604 242
- Dialog Information Services, File 350, World Patent Index 63-80, Dialog Accession no. 2527559, "High yield dialkyl carbonic acid ester preparation - by reacting alkylene carbonate and aliphatic alcohol in presence of catalyst comprising Lewis acid and nitrogen-containing organic bases"; & JP-A-55 064 550

## Beschreibung

Die Erfindung betrifft Guerbetcarbonate, ein Verfahren zu ihrer Herstellung sowie ihre Verwendung als Schmierstoffe.

Ester von gesättigten Fettsäuren mit langkettigen gesättigten Alkoholen weisen aufgrund ihres hohen Molekulargewichtes hervorragende Metallbenetzungs- und Schmiereigenschaften auf. Aufgrund ihrer hohen Stockpunkte kommen die Produkte jedoch nicht für Anwendungen im Bereich niedriger Temperaturen in Betracht [Fat.Sci. Technol. 89, 237 (1987)].

Aus den beiden Schriften DE-A 2335728 und US 4604242 sind Carbonate linearer sowie verzweigter Alkohole wie beispielsweise Hexanol oder 2-Ethylhexanol und deren Einsatz als Schmierstoffe bekannt. Die Stoffe weisen jedoch ein nicht zufriedenstellendes Kälteverhalten und eine zu geringe Viskosität auf.

Wachsester mit einem verbesserten Temperaturverhalten werden auf Basis von ungesättigten Fettsäuren und/oder ungesättigten Fettalkoholen erhalten. Produkte, wie beispielsweise Oleyloleat oder Oleylerucat sind zwar bei niedrigen Temperaturen flüssig, jedoch infolge der im Molekül enthaltenen Doppelbindungen anfällig für oxidative Schädigungen, die im günstigsten Fall zu einer unerwünschten Verfärbung, im schlimmsten Fall zu einer Zersetzung des Produktes führen können.

Die Aufgabe der Erfindung bestand somit darin, neue Schmierstoffe zu entwickeln, die frei von den geschilderten Nachteilen sind.

Gegenstand der Erfindung sind Guerbetcarbonate der Formel **(I)**, 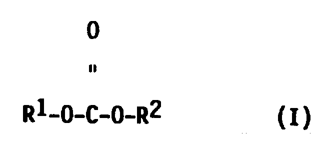 in der R¹ für einen verzweigten Alkylrest mit 12 bis 44 Kohlenstoffatomen und R für R¹ oder einen linearen Alkylrest mit 1 bis 12 Kohlenstoffatomen steht.

Überraschenderweise wurde gefunden, daß die erfindungsgemäßen Guerbetcarbonate nicht nur über gute Schmiereigenschaften verfügen, sondern auch bei tiefen Temperaturen als farblose, blanke Flüssigkeiten vorliegen. Die Erfindung schließt ferner die Erkenntnis ein, daß die Produkte hydrolyse- und oxidationsbeständig sind.

Guerbetcarbonate stellen Diester der Kohlensäure dar. Produkte mit besonders vorteilhaften anwendungstechnischen Eigenschaften liegen vor, wenn R¹ und R in der allgemeinen Formel **(I)** für verzweigte Alkylreste mit 16 bis 20 Kohlenstoffatomen stehen.

Die Erfindung betrifft ferner ein Verfahren zur Herstellung von Guerbetcarbonaten, das sich dadurch auszeichnet, daß man
a) Guerbetalkohole der Formel **(II)**

   **R¹OH (II)**

   in der R¹ für einen verzweigten Alkylrest mit 12 bis 44 Kohlenstoffatomen steht, in Gegenwart von Alkali- oder Erdalkalimetallverbindungen mit Dialkylcarbonaten der Formel **(III)**, 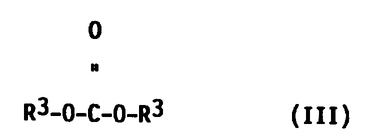 in der R³ für einen linearen Alkylrest mit 1 bis 12 Kohlenstoffatomen steht, bei erhöhter Temperatur umestert und
b) die Umesterungsprodukte mit einem Filterhilfsmittel behandelt und anschließend filtriert.

Verzweigte Fettalkohole vom Typ der Guebetalkohole stellen bekannte Stoffe dar, die nach den einschlägigen Methoden der präparativen organischen Chemie erhalten werden können. Ein klassisches Verfahren zu ihrer Herstellung ist die Kondensation ("Guerbetisierung") von primären linearen Alkoholen in Gegenwart basischer Katalysatoren, die über die Zwischenstufen der Aldehyde und Aldole verläuft **[Soap Cosm.Chem.Spec., 52 (1987)]**.

Guerbetalkohole, die als Ausgangsstoffe für die Herstellung der Guerbetcarbonate in Betracht kommen, können 12 bis 44 Kohlenstoffatome enthalten. Insbesondere werden Guerbetalkohole der allgemeinen Formel **(II)** eingesetzt, bei denen R¹ für einen verzweigten Alkylrest mit 16 bis 20 Kohlenstoffatomen steht. Die bevorzugten Guerbetalkohole sind somit jene, die man durch Guerbetisierung von Fettalkoholen mit 8 bis 10 Kohlenstoffatomen erhält. Es ist ferner möglich, Mischungen verschiedener Guerbetalkohole in die Umesterung einzusetzen.

Auch Dialkylcarbonate stellen bekannte Stoffe dar, die nach den einschlägigen Methoden der präparativen organischen Chemie zugänglich sind. Ein Verfahren zu ihrer Herstellung besteht beispielsweise darin, primäre Alkohole mit Phosgen oder Chlorameisensäureester umzusetzen **[J.Chem.Soc., 117, 708 (1920), J.Prakt.Chem. 22, 353 (1980)]**.

Dialkylcarbonate, die als Ausgangsstoffe für die Herstellung der Guerbetcarbonate in Betracht kommen, sind beispielsweise Dipropylcarbonat, Dibutylcarbonat, Dioctylcarbonat oder Dilaurylcarbonat. Vorzugsweise setzt man jedoch Dialkylcarbonate der Formel **(III)** ein, bei denen R³ für einen Methyl- oder Ethylrest steht. Die bevorzugten Dialkylcarbonate sind also Dimethyl- und Diethylcarbonat.

Die Synthese der erfindungsgemäßen Produkte kann in an sich bekannter Weise durch Umesterung der Dialkylcarbonate mit den Guerbetalkoholen in Gegenwart basischer Katalysatoren durchgeführt werden **[Houben-Weyl, Methoden der Organischen Chemie, 4. Aufl., Bd. E4, S.66 ff.]**.

Als alkalische Katalysatoren kommen dabei Alkali- oder Erdalkalimetallverbindungen, insbesondere Alkali- oder Erdalkalimetallhydroxide, -alkoholate, -carbonate oder -hydrogencarbonate in Betracht. Bevorzugt ist die Verwendung einer Lösung von Natriummethylat in Methanol.

Die Alkali- oder Erdalkalimetallverbindungen können in Mengen von 1 bis 10, vorzugsweise 1 bis 5 Gew.-% - bezogen auf die Guerbetalkohole - in die Umesterung eingesetzt werden.

Die Guerbetalkohole und die Dialkylcarbonate können in molaren Verhältnissen von 1 : 10 bis 10 : 1 eingesetzt werden. Zur Herstellung von symmetrischen Guerbetcarbonaten, also solchen Produkten, die durch Umesterung beider Estergruppen des Dialkylcarbonats erhalten werden, hat es sich als vorteilhaft erwiesen, die Alkoholkomponente im molaren Überschuß einzusetzen. Als optimal wurde ein molares Einsatzverhältnis von 1 : 1 bis 5 : 1, insbesondere 1 : 1 bis 2 : 1 gefunden.

Für die Herstellung von unsymmetrischen Carbonaten, also Produkten, die durch Umesterung von nur einer der beiden Estergruppen des Dialkylcarbonats erhalten werden, wird vorteilhafterweise das Dialkylcarbonat im molaren Überschuß eingesetzt. Als optimal wurde ein molares Einsatzverhältnis von Guerbetalkohol zu Dialkylcarbonat von 1 : 2 bis 1 : 5 gefunden.

Die Umesterung der Dialkylcarbonate kann bei Temperaturen von 80 bis 290°C durchgeführt werden. Als optimal im Hinblick auf die thermische Belastung der Produkte hat es sich als vorteilhaft erwiesen, Reaktionstemperaturen von 80 bis 150°C zu wählen.

Im Verlauf der Umesterung wird der freiwerdende Alkohol kontinuierlich abdestilliert und damit dem Reaktionsgleichgewicht entzogen. Bei Einsatz von Dimethylcarbonat als Ausgangsmaterial bildet sich im Verlauf der Umsetzung ein Azeotrop, welches Methanol und das Dialkylcarbonat enthält und das besonders leicht abgetrennt werden kann. Spuren nicht umgesetzter Ausgangsstoffe oder freigesetzten Alkohols können nachträglich durch Behandlung des Rohproduktes im Vakuum bei Temperaturen unter 150°C abgetrennt werden.

Nach Abschluß der Umesterungsreaktion muß das noch basische Produkt neutralisiert werden. Dies geschieht vorteilhafterweise durch Behandlung mit neutralen oder sauren Filtrationshilfsmitteln, beispielsweise Tonerden oder Schichtsilicaten vom Typ Tonsil^{(R)} oder Celite^{(R)}. Die Filtrationshilfsmittel werden der Reaktionsmischung dabei in Mengen von 0,1 bis 10, vorzugsweise 0,5 bis 2 Gew.-% - bezogen auf die Guerbetcarbonate - zugesetzt. Nach Abtrennung der Zusatzstoffe, beispielsweise durch Filtration oder Zentrifugation, werden die Guerbetcarbonate als praktisch neutrale, oder leicht basische, klare, hellfarbige Flüssigkeiten erhalten.

Die erfindungsgemäßen Guerbetcarbonate sind niedrigviskos, weisen gute Schmiereigenschaften auf und liegen auch bei Temperaturen von - 25°C als blanke Flüssigkeiten vor. Sie eignen sich als Schmierstoffe sowie zur Herstellung von Schmierstoffen.

Die folgenden Beispiele sollen den Gegenstand der Erfindung näher erläutern, ohne ihn darauf einzuschränken.

### Beispiele

### Beispiel 1:

**Bis-C₁₆-Guerbetcarbonat.** In einem 2-l-Dreihalskolben mit Rührer, Innenthermometer und Destillationsaufsatz wurden 1666 g (6 mol) eines C₁₆-Guerbetalkohols (Gerbitol^{(R)} G16, Hydroxylzahl = 202, Verseifungszahl = 6,2, Fa. Henkel KGaA) vorgelegt und mit 446 g (4,95 mol) Dimethylcarbonat und 38 g einer 30 gew.-%igen Lösung von Natriummethylat in Methanol versetzt. Die Mischung wurde unter Stickstoff zunächst über einen Zeitraum von 1 h auf 80°C erhitzt. Anschließend wurde die Reaktionstemperatur auf 95°C erhöht und über einen Zeitraum von 5 h bis auf 150°C gesteigert, wobei ein Azeotrop enthaltend überschüssiges Dimethylcarbonat und Methanol abdestillierte. Im Anschluß wurde das gebildete rohe Guerbetcarbonat im Wasserstrahlvakuum bei Temperaturen unter 150°C vom restlichen Methanol befreit. Schließlich wurde das Produkt mit 30 g, entsprechend 1,5 Gew.-% Tonsil^{(R)} - bezogen auf das gebildete Guerbetcarbonat - verrührt und durch Filtration gereinigt. Das Bis-C16-Guerbetcarbonat wurde in Form einer klaren Flüssigkeit gewonnen. Die Ausbeute betrug 97 % der theoretischen Menge.

| Kenndaten des Produktes: | |
|---|---|
| Hydroxylzahl | : 10,8 |
| Pourpoint | : < - 50°C |
| Cloudpoint | : < - 50°C |
| Viskosität | : 37 mPas |

### Beispiel 2:

**Bis-C₂₀-Guerbetcarbonat.** Analog zu Beispiel 1 wurden 1690 (5 mol C₂₀-Guerbetalkohol (Guerbitol^{(R)} G20, Hydroxylzahl 166, Verseifungszahl = 5, Fa. Henkel KGaA) mit 371 g (4,12 mol) Dimethylcar bonat und 32 g einer 30 gew.-%igen Lösung von Natriummethylat in Methanol umgesetzt. Das Bis-C₂₀-Guerbetcarbonat wurde in Form einer klaren Flüssigkeit gewonnen. Die Ausbeute betrug 96 % der theoretischen Menge.

| Kenndaten des Produktes: | |
|---|---|
| Hydroxylzahl | : 5,7 |
| Pourpoint | : - 25°C |
| Cloudpoint | : - 28°C |
| Viskosität | : 62 mPas |

Pourpoint und Cloudpoint wurden gemäß **DIN 51 601** bzw. **DIN ISO 3015** bestimmt. Die Messung der Viskosität erfolgte in einem Höppler-Viskosimeter.

## Patentansprüche

1. Guerbetcarbonate der Formel **(I)**, 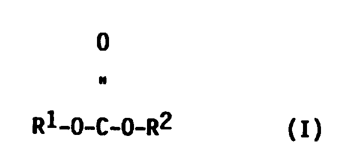 in der R¹ für einen verzweigten Alkylrest mit 12 bis 44 Kollenstoffatomen und R für R¹ oder einen linearen Alkylrest 1 bis 12 Kohlenstoffatomen steht.

2. Verfahren zur Herstellung von Guerbetcarbonaten, **dadurch gekennzeichnet,** daß man
a) Guerbetalkohole der Formel **(II)**
**R¹OH (II)**
in der R¹ für einen verzweigten Alkylrest mit 12 bis 44 Kohlenstoffatomen steht, in Gegenwart von Alkali- oder Erdalkalimetallverbindungen mit Dialkylcarbonaten der Formel **(III)**, 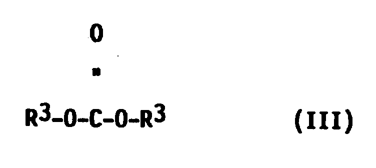 in der R³ für lineare Alkylreste mit 1 bis 12 Kohlenstoffatomen steht, bei erhöhter Temperatur umestert und
b) die Umesterungsprodukte mit einem Filterhilfsmittel behandelt und anschließend filtriert.

3. Verfahren nach Anspruch 2, **dadurch gekennzeichnet,** daß man Guerbetalkohole der Formel **(II)** einsetzt, bei denen R¹ für einen verzweigten Alkylrest mit 16 bis 20 Kohlenstoffatomen steht.

4. Verfahren nach mindestens einem der Ansprüche 2 und 3, **dadurch gekennzeichnet,** daß man Dialkylcarbonate der Formel **(III)** einsetzt, bei denen R³ für einen Methyl- oder Ethylrest steht.

5. Verfahren nach mindestens einem der Ansprüche 2 bis 4, **dadurch gekennzeichnet,** daß man als Alkali- oder Erdalkalimetallverbindungen Alkali- oder Erdalkalimetallhydroxide, -alkoholate, -carbonate oder -hydrogencarbonate einsetzt.

6. Verfahren nach mindestens einem der Ansprüche 2 bis 5, **dadurch gekennzeichnet,** daß man die Alkali- oder Erdalkalimetallverbindungen in Mengen von 1 bis 10 Gew.-% - bezogen auf die Guerbetalkohole - einsetzt.

7. Verfahren nach mindestens einem der Ansprüche 2 bis 6, **dadurch gekennzeichnet,** daß man die Guerbetalkohole und die Dialkylcarbonate in molaren Verhältnissen von 1 : 10 bis 10 : 1 einsetzt.

8. Verfahren nach mindestens einem der Ansprüche 2 bis 7, **dadurch gekennzeichnet,** daß man die Umesterung bei Temperaturen von 80 bis 290°C durchführt.

9. Verfahren nach mindestens einem der Ansprüche 2 bis 8, **dadurch gekennzeichnet,** daß man die Filterhilfsmittel in Mengen von 0,1 bis 10 Gew.-% - bezogen auf die Guerbetcarbonate - einsetzt.

10. Verfahren nach mindestens einem der Ansprüche 2 bis 9, **dadurch gekennzeichnet,** daß man als Filterhilfsmittel Tonerden oder Schichtsilicate einsetzt.

11. Verwendung von Guerbetcarbonaten nach Anspruch 1 als Schmierstoffe.

12. Verwendung von Guerbetcarbonaten erhältlich nach dem Verfahren nach mindestens einem der Ansprüche 2 bis 10 als Schmierstoffe.

## Claims

1. Guerbet carbonates corresponding to formula **(I)** 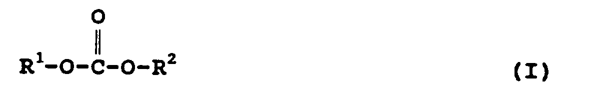 in which R¹ is a branched alkyl radical containing 12 to 44 carbon atoms and R has the same meaning as R¹ or is a linear alkyl radical containing 1 to 12 carbon atoms.

2. A process for the production of Guerbet carbonates, **characterized in that**
a) Guerbet alcohols corresponding to formula **(II)**
**R¹OH (II)**
in which R¹ is a branched alkyl radical containing 12 to 44 carbon atoms, are transesterified at elevated temperature with dialkyl carbonates corresponding to formula **(III)** 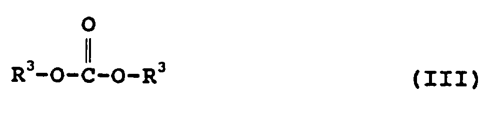 in which R³ is a linear alkyl radical containing 1 to 12 carbon atoms, in the presence of alkali metal or alkaline earth metal compounds and
b) the transesterification products are treated with a filter aid and subsequently filtered.

3. A process as claimed in claim 2, **characterized in that** Guerbet alcohols corresponding to formula **(II)**, in which R¹ is a branched alkyl radical containing 16 to 20 carbon atoms, are used.

4. A process as claimed in at least one of claims 2 and 3, **characterized in that** dialkyl carbonates corresponding to formula **(III)**, in which R³ is a methyl or ethyl radical, are used.

5. A process as claimed in at least one of claims 2 to 4, **characterized in that** alkali metal or alkaline earth metal hydroxides, alcoholates, carbonates or hydrogen carbonates are used as the alkali metal or alkaline earth metal compounds.

6. A process as claimed in at least one of claims 2 to 5, **characterized in that** the alkali metal or alkaline earth metal compounds are used in quantities of 1 to 10% by weight, based on the Guerbet alcohols.

7. A process as claimed in at least one of claims 2 to 6, **characterized in that** the Guerbet alcohols and dialkyl carbonates are used in molar ratios of 1:10 to 10:1.

8. A process as claimed in at least one of claims 2 to 7, **characterized in that** the transesterification is carried out at temperatures of 80 to 290°C.

9. A process as claimed in at least one of claims 2 to 8, **characterized in that** the filter aids are used in quantities of 0.1 to 10% by weight, based on the Guerbet carbonates.

10. A process as claimed in at least one of claims 2 to 9, **characterized in that** aluminas or layer silicates are used as filter aids.

11. The use of the Guerbet carbonates claimed in claim 1 as lubricants.

12. The use of the Guerbet carbonates obtainable by the process claimed in at least one of claims 2 to 10 as lubricants.

## Revendications

1. Carbonates de guerbet de formule (I): 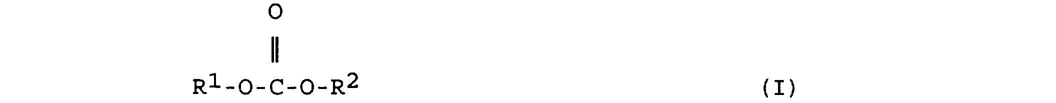 où R¹ est un radical alkyle ramifié de 22 à 44 atomes de carbone et R pour R¹ ou un radical alkyle linéaire de 1 à 12 atomes de carbone.

2. Procédé de préparation de carbonates de guerbet, caractérisé en ce qu'on trans-estérifie à haute température :
a) des alcools de guerbet de formule (II)
R¹OH (II)
où R¹ est un radical alkyle ramifié de 12 à 44 atomes de carbone, en présence de composés de métal alcalin ou alcalino-terreux avec des dialkylcarbonates de formule (III) : 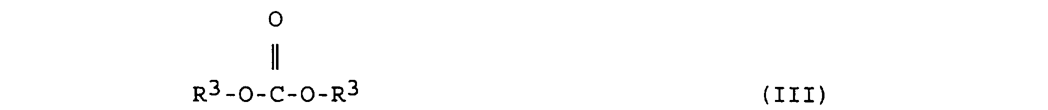 où R³ représente des restes alkyles de 1 à 12 atomes de carbone, et
b) on traite les produits de trans-estérification avec un adjuvant de filtration et ensuite on filtre.

3. Procédé selon la revendication 2, caractérisé en ce qu'on utilise des alcools de guerbet de formule (II), où R¹ est un radical alkyle ramifié de 16 à 20 atomes de carbone.

4. Procédé selon au moins l'une des revendications 2 et 3, caractérisé en ce qu'on utilise des carbonates de dialkyle de formule (III), où R³ est un radical méthyle ou éthyle.

5. Procédé selon au moins l'une des revendications 2 à 4, caractérisé en ce qu'on utilise comme composés de métal alcalin ou alcalin terreux des hydroxydes, alcoolates, carbonates et hydrogénocarbonates de métal alcalin ou alcalino-terreux.

6. Procédé selon au moins l'une des revendications 2 à 5, caractérisé en ce qu'on utilise des composés de métal alcalin ou alcalino-terreux en des quantités de 1 à 10% en poids par rapport aux alcools de guerbet.

7. Procédé selon au moins l'une des revendications 2 à 6, caractérisé en ce qu'on utilise les alcools de guerbet et les carbonates de dialkyle dans des rapports molaires de 1:10 à 10:1.

8. Procédé selon au moins l'une des revendications 2 à 7, caractérisé en ce qu'on réalise la trans-estérification à des températures de 80 à 290°C.

9. Procédé selon au moins l'une des revendications 2 à 8, caractérisé en ce qu'on utilise les adjuvants de filtration en des quantités de 0,1 à 10% en poids par rapport aux carbonates de guerbet.

10. Procédé selon au moins l'une des revendications 2 à 9, caractérisé en ce qu'on utilise comme adjuvants de filtration de l'alumine ou des silicates feuilletés.

11. Utilisation de carbonates de guerbet selon la revendication 1 comme lubrifiants.

12. Utilisation comme lubrifiants de carbonates de guerbet qu'on peut obtenir selon le procédé d'au moins l'une des revendications 2 à 10.
